# EUROPÄISCHE PATENTSCHRIFT

(11) **EP 2 321 254 B1**
(45) Veröffentlichungstag und Bekanntmachung des Hinweises auf die Patenterteilung: **30.07.2014**
(21) Anmeldenummer: 09782586.3
(22) Anmeldetag: 04.09.2009
(51) Int. Cl.: C07C 69/54, C07C 67/293, C08F 220/18, C08F 220/26, C09D 167/08, C09D 133/14, C08L 33/14, C08F 8/00, C08F 220/28, C08F 220/14, C08F 220/06

(54) **FUNKTIONALISIERTES (METH) ACRYLATMONOMER, POLYMER, BESCHICHTUNGSMITTEL UND VERFAHREN ZUR HERSTELLUNG UND VERNETZUNG**
FUNCTIONALIZED (METH)ACRYLATE MONOMER, POLYMER, COATING AGENT, AND PRODUCTION AND CROSS-LINKING METHOD
MONOMÈRE DE (MÉTH)ACRYLATE FONCTIONNALISÉ, POLYMÈRE, PRODUIT DE REVÊTEMENT ET PROCÉDÉS ASSOCIÉS DE PRÉPARATION ET DE RÉTICULATION

(30) Priorität: 08.09.2008 DE 102008046076; 08.06.2009 DE 102009026820
(43) Veröffentlichungstag der Anmeldung: 18.05.2011
(73) Patentinhaber: Evonik Röhm GmbH, 64293 Darmstadt (DE)
(72) Erfinder: SCHÜTZ, Thorben, 64342 Seeheim-Jugenheim (DE); KNEBEL, Joachim, 64665 Alsbach-Hähnlein (DE); GOMEZ ANDREU, Mario, 64319 Pfungstadt (DE); BREINER, Christine Maria, 69514 Laudenbach (DE); JACKSTELL, Ralf, 18106 Rostock (DE); BELLER, Matthias, 18211 Nienhagen (DE); GROTEVENDT, Anne, 17139 Malchin (DE); FRIDAG, Dirk, 45721 Haltern am See (DE); FRANKE, Robert, 45772 Marl (DE)
(86) Internationale Anmeldenummer: PCT/EP2009/061429
(87) Internationale Veröffentlichungsnummer: WO 2010/026204

(56) Entgegenhaltungen:
- EP-A- 0 003 516
- EP-A- 1 174 411
- EP-A2- 0 422 888
- WO-A-2008/062900
- WO-A1-2009/024216
- DE-A1- 2 207 209
- DE-A1- 2 722 097
- DE-A1- 4 219 384
- DE-A1-102007 039 312
- US-A- 4 191 838
- US-A- 5 068 264
- US-A- 5 800 733
- US-A1- 2002 187 282
- US-A1- 2003 028 048
- ANTONUCCI: "Aldehyde Methacrylates Derived from Hydroxybenzaldehydes" JOURNAL OF DENTAL RESEARCH, Bd. 57, Nr. 3, 1978, Seiten 500-505, XP002553557
- ZABRANSKY ET AL.: "Aldehydes and acetals based on acryl- and methacrylamides as potential monomers" MAKROMOLEKULARE CHEMIE, Bd. 186, 1985, Seiten 223-229, XP008114207
- ZABRANSKY ET AL.: "Aldehydes and acetals based on esters of methacrylic acid as potential monomers" MAKROMOLEKULARE CHEMIE, Bd. 186, 1985, Seiten 215-222, XP008114208
- MION ET AL.: "Procédé de déracémisation des acides alpha-aminés via le pyridoxal. I. Synthèse et activité de formes polymérisables du pyridoxal" BULL. SOC. CHIM. FR., Bd. 132, 1995, Seiten 709-720, XP002553558
- FUKUMOTO ET AL.: "Stereocontrolled Construction of Octahydroquinolizines, Octahydroindolizines, Hexahydrobenzo[a]quinolizines, and an Octahydroindolo[2,3-a]quinolizine by an Intramolecular Double Michael Reaction: Synthesis of (+/-)-Epilupinine" J. CHEM. SOC. PERKIN TRANS. I, 1987, Seiten 1719-1726, XP002553559
- SHARMA ET AL.: "RCM mediated synthesis of macrosphelides I and G" TETRAHEDRON ASYMMETRY, Bd. 18, 2007, Seiten 2175-2184, XP002553560
- CAYE F ET AL: "A CONVENIENT HIGH YIELD SYNTHESIS OF FUNCTIONAL METHACRYLATES VIA DETHIOACETALIZATION. SYNTHESIS OF METHACRYLATE S,S-ACETAL DERIVATIVES AS INTERMEDIATES" PHOSPHORUS, SULFUR AND SILICON AND THE RELATED ELEMENTS, GORDON AND BREACH SCIENCE PUBLISHERS, AMSTERDAM, GB, Bd. 143, 1. Januar 1998 (1998-01-01), Seiten 197-220, XP008032371 ISSN: 1042-6507

## Beschreibung

Die vorliegende Erfindung betrifft ein Verfahren zur Herstellung eines funktionalisierten (Meth)acrylatmonomers.

Beschichtungsmittel, insbesondere Lacke werden seit langer Zeit synthetisch hergestellt. Neuere Beschichtungsmittel umfassen Carbonylgruppen-haltige Polymere, die durch Zugabe von Vernetzungsmitteln zu relativ lösungsmittelbeständigen Lacken gehärtet werden können. Diese Beschichtungsmittel werden unter anderem in WO 94/025433 dargelegt. In der DE 4219384 werden Dispersionen oder Lösungen eines radikalischen Copolymerisats offenbart, welches Aldehydgruppen enthält. Besagte Dispersionen oder Lösungen werden als Beschichtungsmittel verwendet. Für die Copolymerisate werden als Monomere (Meth)acryloxyalkylpropanale genannt, welche in der DE 2722097 beschrieben werden. Die DE 2722097 beschreibt eine Synthese durch Umsetzung von (Meth)acrylsäure mit Hydroxyalkylpropanalen. Allerdings ist die Verbesserung des Eigenschaftsprofils dieser Beschichtungsmittel ein dauerhaftes Bedürfnis.

In Anbetracht des Standes der Technik ist es nun Aufgabe der vorliegenden Erfindung Monomere zur Verfügung zu stellen, die sich zu Polymeren mit hervorragenden Eigenschaften verarbeiten lassen. Zu diesen Eigenschaften gehören insbesondere Merkmale, die durch Beschichtungsmittel und Beschichtungen, die aus den Beschichtungsmitteln erhältlich sind, offenbar werden.

Insbesondere sollten sich die Monomere zu Dispersionen bzw. zu Polymeren, beispielsweise Emulsionspolymeren, verarbeiten lassen, die einen sehr geringen Restmonomergehalt aufweisen.

Eine weitere Aufgabe kann darin gesehen werden, ein Verfahren zur Herstellung dieser Monomere bereitzustellen, bei dem das Produkt sehr kostengünstig erhalten werden kann. Darüber hinaus sollte das erhaltene Monomere nur sehr geringe Mengen an Nebenprodukten und Katalysatorresten enthalten.

Eine weitere Aufgabe der Erfindung bestand darin, ein Verfahren zu schaffen, bei dem das Monomer sehr selektiv erhalten werden kann. Hierbei sollten die gemäß dem vorliegenden Verfahren erhältlichen Monomere problemlos in weiteren Verfahrensschritten umgesetzt werden können, ohne dass eine aufwendige Aufreinigung notwendig sein sollte.

Darüber hinaus war es Aufgabe der vorliegenden Erfindung, Verfahren zur Herstellung von Monomeren zur Verfügung zu stellen, die einfach und kostengünstig durchgeführt werden können. Hierbei sollte das Produkt möglichst in hohen Ausbeuten und, insgesamt gesehen, unter geringem Energieverbrauch erhalten werden.

Gelöst werden diese sowie weitere nicht explizit genannte Aufgaben, die jedoch aus den hierin einleitend diskutierten Zusammenhängen ohne weiteres ableitbar oder erschließbar sind, durch ein Verfahren mit allen Merkmalen des Patentanspruchs 1. Zweckmäßige Abwandlungen des erfindungsgemäßen Verfahrens werden in Unteransprüchen unter Schutz gestellt.

Gegenstand der vorliegenden Erfindung ist dementsprechend ein Verfahren zur Herstellung von(Meth)acrylatmonomeren der allgemeinen Formel (I) 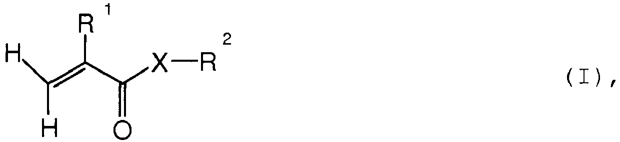 worin R¹ Wasserstoff oder eine Methylgruppe, X Sauerstoff oder eine Gruppe der Formel NR', worin R' Wasserstoff oder ein Rest mit 1 bis 6 Kohlenstoffatomen ist, R² ein Rest mit 3 bis 31 Kohlenstoffatomen und mindestens einer Aldehydgruppe ist.

Darüber hinaus stellt die vorliegende Erfindung ein Verfahren zur Herstellung von funktionalisierten (Meth)acrylaten zur Verfügung, bei dem das Monomer sehr kostengünstig erhalten wird. Überraschend enthält das erhaltene (Meth)acrylat nur sehr geringe Mengen an Nebenprodukten, wobei im Allgemeinen keine Katalysatorreste in der Produktmischung enthalten sind. Dementsprechend kann eine gemäß dem vorliegenden Verfahren erhältliche Zusammensetzung problemlos in weiteren Verfahrensschritten umgesetzt werden, ohne dass eine aufwendige Aufreinigung notwendig ist.

Des Weiteren ermöglicht das erfindungsgemäße Verfahren eine besonders selektive Herstellung von funktionalisierten (Meth)acrylaten.

Weiterhin kann das erfindungsgemäße Verfahren einfach und kostengünstig durchgeführt werden, wobei das Produkt in hohen Ausbeuten und, insgesamt gesehen, unter geringem Energieverbrauch erhalten werden kann.

Das (Meth)acrylatmonomer entspricht der allgemeinen Formel (I) 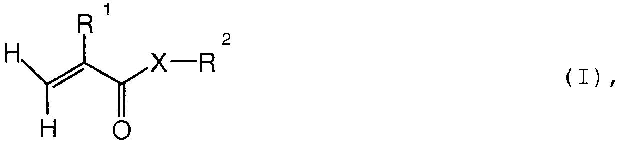 worin R¹ Wasserstoff oder eine Methylgruppe, X Sauerstoff oder eine Gruppe der Formel NR', worin R' Wasserstoff oder ein Rest mit 1 bis 6 Kohlenstoffatomen ist, R² ein Rest mit 3 bis 31 Kohlenstoffatomen und mindestens einer Aldehydgruppe ist.

Der Ausdruck "Rest mit 1 bis 6 Kohlenstoffatomen" bzw. "Rest mit 3 bis 31 Kohlenstoffatomen" steht für eine Gruppe, die 1 bis 6 bzw. 3 bis 31 Kohlenstoffatome aufweist. Er umfasst aromatische und heteroaromatische Gruppen sowie Alkyl-, Cycloalkyl-, Alkoxy-, Cycloalkoxy-, Alkenyl-, Alkanoyl-, Alkoxycarbonylgruppen sowie heteroalipatische Gruppen. Dabei können die genannten Gruppen verzweigt oder nicht verzweigt sein. Des Weiteren können diese Gruppen Substituenten, insbesondere Halogenatome oder Hydroxygruppen aufweisen.

Vorzugsweise stehen die Reste R', für Alkylgruppen. Zu den bevorzugten Alkylgruppen gehören die Methyl-, Ethyl-, Propyl-, Isopropyl-, 1-Butyl-, 2-Butyl-, 2-Methylpropyl-, tert.-Butyl-Gruppe.

In Formel bedeutet der Rest R² eine Gruppe mit 3 bis 31 Kohlenstoffatomen, insbesondere mit 3 bis 25, vorzugsweise mit 3 bis 9, besonders bevorzugt mit 4 bis 6 Kohlenstoffatomen, der mindestens eine Aldehydgruppe umfasst. Gemäß einer weiteren Ausgestaltung der vorliegenden Erfindung sind (Meth)acrylatmonomere bevorzugt, die 10 bis 25 Kohlenstoffatome aufweisen. Hierbei kann der Rest R² ein, zwei, drei oder mehr Aldehydgruppen umfassen, wobei der Rest R² substituiert sein kann und weitere funktionale Gruppen, beispielsweise C-C-Doppelbindungen enthalten kann. Gemäß einer bevorzugten Abwandlung der vorliegenden Erfindung stellt der Rest R² eine Alkyl- oder Alkenylgruppe dar, die eine oder zwei Aldehydgruppen umfasst, wobei Reste mit genau einer Aldehydgruppe besonders bevorzugt sind. Diese Gruppe kann Heteroatome, insbesondere Sauerstoff- und/oder Stickstoffatome, beispielsweise als Ester-, Ether-, Amino- und/oder Amidgruppe umfassen.

Zu den bevorzugten Resten R² zählt insbesondere die 2-Formylethyl-, 3-Formylethyl-, 2-Formylpropyl-, 3-Formylpropyl-, 2-Formyl-octa-7-enyl-, 2,7-Diformyloctyl-, 9-Formyloctadecyl- und 10- Formyloctadecyl-Gruppe.

Zu den bevorzugten (Meth)acrylatmonomeren gemäß Formel (I) gehören unter anderem (Meth)acrylatmonomere mit 3 bis 9 Kohlenstoffatomen im Rest R², wie zum Beispiel 3-Oxopropyl(meth)acrylat (2-Formylethyl(meth)acrylat), 4-Oxobutyl(meth)acrylat (3-Formylpropyl(meth)acrylat), 2-Methyl-3-oxopropyl(meth)acrylat (2-Formyl-2-methylethyl(meth)acrylat), 2-Formyl-octenyl(meth)acrylat, 3-Formyl-octenyl(meth)acrylat, 8-Formyl-octenyl(meth)acrylat, 7-Formyl-octenyl(meth)acrylat, 2,8-Diformyloctyl(meth)acrylat und 3,7-Diformyloctyl(meth)acrylat.

Darüber hinaus gehören zu den (Meth)acrylatmonomeren gemäß Formel (I) (Meth)acrylatmonomere mit 10 bis 25 Kohlenstoffatomen im Rest R², wie (Meth)acrylate, die sich von Fettsäuren, Fettalkoholen und Fettsäureamiden ableiten, wie 9-Formyloctadecan-12-en-yl-(meth)acrylat, 9,12-Diformyloctadecyl-(meth)acrylat, 12-Formyloctadecan-6,9-dien-yl-(meth)acrylat, 9-Formylhexadecyl(meth)acrylat, 10-Formylhexadecyl(meth)acrylat, 9-Formyloctadecyl(meth)acrylat, 10-Formyloctadecyl(meth)acrylat, (Meth)acryloyloxy-2-hydroxypropyl-9-formyloctadecansäureester, (Meth)acryloyloxy-2-hydroxypropyl-10-formyloctadecansäureester, (Meth)acryloyloxy-2-hydroxypropyl-9-formyloctadecansäureamid und/oder (Meth)acryloyloxy-2-hydroxypropyl-10-formyloctadecansäureamid.

Darüber hinaus gehören zu den (Meth)acrylatmonomeren gemäß Formel (I) (Meth)acrylate, die der Formel (II) entsprechen 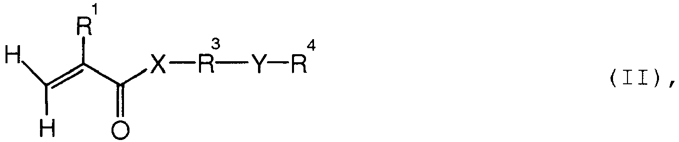 worin R¹ Wasserstoff oder eine Methylgruppe, X Sauerstoff oder eine Gruppe der Formel NR', worin R' Wasserstoff oder ein Rest mit 1 bis 6 Kohlenstoffatomen ist, R³ eine Alkylengruppe mit 1 bis 22 Kohlenstoffatomen, Y Sauerstoff, Schwefel oder eine Gruppe der Formel NR" bedeutet, worin R" Wasserstoff oder ein Rest mit 1 bis 6 Kohlenstoffatomen darstellt, und R⁴ ein Rest mit 8 Kohlenstoffatomen und mindestens einer Aldehydgruppe ist.

Vorzugsweise stehen die Reste R', R " für Alkylgruppen. Zu den bevorzugten Alkylgruppen gehören die Methyl-, Ethyl-, Propyl-, Isopropyl-, 1-Butyl-, 2-Butyl-, 2-Methylpropyl-, tert.-Butyl-Gruppe.

In Formel bedeutet der Rest R³ eine Alkylengruppe mit 1 bis 22 Kohlenstoffatomen, vorzugsweise mit 1 bis 10, besonders bevorzugt mit 2 bis 6 Kohlenstoffatomen. Der Rest R³ stellt gemäß einer besonderen Ausführungsform der vorliegenden Erfindung eine Alkylengruppe mit 2 bis 4, besonders bevorzugt 2 Kohlenstoffatomen dar. Zu den Alkylengruppen mit 1 bis 22 Kohlenstoffatomen gehören insbesondere die Methylen-, Ethylen-, Propylen-, iso-Propylen-, n-Butylen-, iso-Butylen-, t-Butylen- oder Cyclohexylen-Gruppe, wobei die Ethylengruppe besonders bevorzugt ist.

Der Rest R⁴ umfasst mindestens eine Aldehydgruppe, vorzugsweise zwei Aldehydgruppen. Gemäß einem weiteren Aspekt stellt der Rest R⁴ eine Gruppe mit einer Aldehydgruppe und einer Doppelbindung dar.

Zu den (Meth)acrylaten gemäß Formel (II) gehören zum Beispiel 2-[(2-Formylocta-7-enyl)methylamino]ethyl-2-methylprop-2-enoat; 2-[(7-Formylocta-2-enyl)methylamino]ethyl-2-methylprop-2-enoat; 2-[(3-Formylocta-7-enyl)methylamino]ethyl-2-methylprop-2-enoat; 2-[(8-Formylocta-2-enyl)methylamino]ethyl-2-methylprop-2-enoat; 2-[(2,7-Diformyloctyl)methylamino]ethyl-2-methylprop-2-enoat; 2-[(3,7-Diformyloctyl)methylamino]ethyl-2-methylprop-2-enoat; 2-[(2,8-Diformyloctyl)methylamino]ethyl-2-methylprop-2-enoat; 2-[(3,8-Diformyloctyl)methylamino]ethyl-2-methylprop-2-enoat; 2-[(2-Formylocta-7-enyl)methylamino]ethyl-(meth)acrylsäureamid; 2-[(7-Formylocta-2-enyl)methylamino]ethyl-(meth)acrylsäureamid; 2-[(3-Formylocta-7-enyl)methylamino]ethyl-(meth)acrylsäureamid; 2-[(8-Formylocta-2-enyl)methylamino]ethyl-(meth)acrylsäureamid; 2-[(2,7-Diformyloctyl)methylamino]ethyl-(meth)acrylsäureamid; 2-[(3,7-Diformyloctyl)methylamino]ethyl-(meth)acrylsäureamid; 2-[(2,8-Diformyloctyl)methylamino]ethyl-(meth)acrylsäureamid; 2-[(3,8-Diformyloctyl)methylamino]ethyl-(meth)acrylsäureamid; (Meth)acryloyloxy-2-hydroxypropyl-9-formyloctadeca-12-ensäureester; (Meth)acryloyloxy-2-hydroxypropyl-12-formyloctadeca-9-ensäureester; (Meth)acryloyloxy-2-hydroxypropyl-10-formyloctadeca-12-ensäureester; (Meth)acryloyloxy-2-hydroxypropyl-13-formyloctadeca-9-ensäureester; (Meth)acryloyloxy-2-hydroxypropyl-9,12-diformyloctadecansäureester; (Meth)acryloyloxy-2-hydroxypropyl-10,13-diformyloctadecansäureester; (Meth)acryloyloxy-2-hydroxypropyl-9-formyloctadecansäureester; (Meth)acryloyloxy-2-hydroxypropyl-10-formyloctadecansäureester; (Meth)acryloyloxy-2-hydroxypropyl-9-formyloctadeca-12-ensäureamid; (Meth)acryloyloxy-2-hydroxypropyl-12-formyloctadeca-9-ensäureamid; (Meth)acryloyloxy-2-hydroxypropyl-10-formyloctadeca-12-ensäureamid; (Meth)acryloyloxy-2-hydroxypropyl-13-formyloctadeca-9-ensäureamid; (Meth)acryloyloxy-2-hydroxypropyl-9,12-diformyloctadecansäureamid; (Meth)acryloyloxy-2-hydroxypropyl-10,13-diformyloctadecansäureamid; (Meth)acryloyloxy-2-hydroxypropyl-9-formyloctadecansäureamid und/oder (Meth)acryloyloxy-2-hydroxypropyl-10-formyloctadecansäureamid.

Die genannten Monomere können einzeln oder als Mischung von zwei oder mehr Verbindungen eingesetzt werden.

(Meth)acrylatmonomeren gemäß Formel (I) lassen sich mit Vorteilen, die für den Fachmann nicht vorhersehbar waren, durch eine Umsetzung eines Edukts der Formel (III) 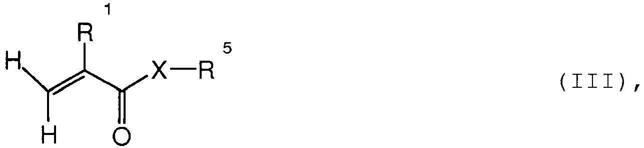 worin X Sauerstoff oder eine Gruppe der Formel NR', worin R' Wasserstoff oder ein Rest mit 1 bis 6 Kohlenstoffatomen ist, R⁵ ein ungesättigter Rest mit mindestens einer Doppelbindung und 2 bis 30 Kohlenstoffatomen, vorzugsweise 2 bis 24 Kohlenstoffatomen ist, mit Kohlenmonoxid und Wasserstoff in Gegenwart eines Katalysators, erhalten.

Umsetzung von ungesättigten Verbindungen mit Kohlenmonoxid und Wasserstoff in Gegenwart eines Katalysators werden vielfach als Hydroformylierungsverfahren bezeichnet. Zu den bevorzugten Katalysatoren zählen insbesondere Verbindungen, die Rhodium, Iridium, Palladium und/oder Cobalt umfassen, wobei Rhodium besonders bevorzugt ist.

Gemäß einer besonderen Ausführungsform können insbesondere Komplexe, die mindestens eine phosphorhaltige Verbindung als Ligand umfassen, zur Katalyse eingesetzt werden. Bevorzugte phosphorhaltige Verbindungen umfassen aromatische Gruppen und mindestens ein, besonders bevorzugt zwei Phosphoratome. Zu den phosphorhaltigen Verbindungen zählen insbesondere Phosphine, Phosphite, Phosphinite, Phosphonite. Beispiele für Phosphine sind Triphenylphosphin, Tris(p-tolyl)phosphin, Tris(m-tolyl)phosphin, Tris(o-tolyl)phosphin, Tris(p-methoxyphenyl)phosphin, Tris(p-dimethylaminophenyl)phosphin, Tricyclohexylphosphin, Tricyclopentylphosphin, Triethylphosphin, Tri-(1-naphthyl)phosphin, Tribenzylphosphin, Tri-n-butylphosphin, Tri-t-butylphosphin. Beispiele für Phosphite sind Trimethylphosphit, Triethylphosphit, Tri-n-propylphosphit, Tri-i-propylphosphit, Tri-n-butylphosphit, Tri-i-butylphosphit, Tri-t-butylphosphit, Tris(2-ethylhexyl)phosphit, Triphenylphosphit, Tris(2,4-di-t-butylphenyl)phosphit, Tris(2-t-butyl-4-methoxyphenyl)phosphit, Tris(2-t-butyl-4-methylphenyl)phosphit, Tris(p-kresyl)phosphit. Beispiele für Phosphonite sind Methyldiethoxyphosphin, Phenyldimethoxyphosphin, Phenyldiphenoxyphosphin, 2-Phenoxy-2H-dibenz[c,e][1,2]oxaphosphorin und dessen Derivate, in denen die Wasserstoffatome ganz oder teilweise durch Alkyl- und/oder Arylreste oder Halogenatome ersetzt sind. Gängige Phosphinitliganden sind Diphenyl(phenoxy)phosphin und dessen Derivate Diphenyl(methoxy)phosphin und Diphenyl(ethoxy)phosphin.

Zu den besonders bevorzugten Liganden zählen insbesondere 4,5-Bis(diphenylphosphino)-9,9-dimethylxanthen (Xantphos) und dessen Derivat 10,10'-(2,7-Di-tert-butyl-9,9-dimethyl-9H-xanthen-4,5-diyl)bis(10H-phenoxaphosphinin) (POP-Xantphos) und Biphephos

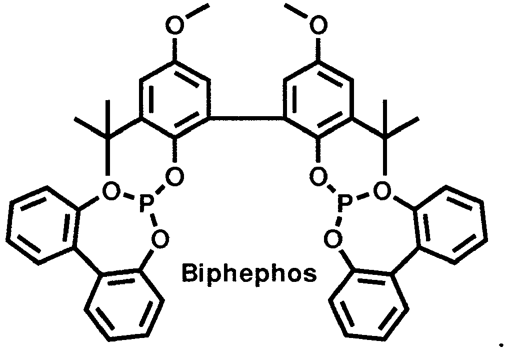

Katalysatoren und Liganden zur Hydroformylierung werden beispielsweise in WO 2008/071508 A1, eingereicht am 13.11.2007 beim Europäischen Patentamt mit der Anmeldenummer PCT/EP2007/062248; EP 982 314 B1, eingereicht am 17.08.1999 beim Europäischen Patentamt mit der Anmeldenummer 99116208; WO 2008/012128 A1 eingereicht am 29.05.2007 beim Europäischen Patentamt mit der Anmeldenummer PCT/EP2007/055165; WO 2008/006633 A1 eingereicht 11.29.05.2007 beim Europäischen Patentamt mit der Anmeldenummer PCT/EP2007/054576; WO 2007/036424 A1 eingereicht 08.09.2006 beim Europäischen Patentamt mit der Anmeldenummer PCT/EP2006/066181; WO 2007/028660 A1 eingereicht 02.06.2006 beim Europäischen Patentamt mit der Anmeldenummer PCT/EP2006/062872; WO 2005/090276 A1 eingereicht 27.01.2005 beim Europäischen Patentamt mit der Anmeldenummer PCT/EP2005/050347 dargelegt, wobei auf diese Druckschriften zu Offenbarungszwecken verwiesen wird und die darin offenbarten Katalysatoren und Liganden in diese Anmeldung eingefügt werden.

Gemäß einer besonderen Ausgestaltung des vorliegenden Verfahrens kann die als Ligand eingesetzte phosphorhaltige Verbindung im Überschuss zum Metall eingesetzt werden. Durch diese Ausgestaltung können überraschende Vorteile hinsichtlich der Selektivität und der Reaktivität erzielt werden. Vorzugsweise kann das Verhältnis von Metall zu Ligand im Bereich von 1:1 bis 1:1000, besonders bevorzugt im Bereich von 1:2 und 1:200 liegen.

Zu den bevorzugten Ausgangsstoffen, die zur Herstellung der (Meth)acrylate gemäß Formel (I) eingesetzt werden können und obiger Formel (II) entsprechen, gehören unter anderem (Meth)acrylate mit 2 bis 8 Kohlenstoffatomen im Alkylrest, die sich von ungesättigten Alkoholen ableiten, und (Meth)acrylate mit 9 bis 24 Kohlenstoffatomen im Alkylrest, die mindestens eine Doppelbindung aufweisen.

Zu den (Meth)acrylate mit 2 bis 8 Kohlenstoffatomen im Alkylrest, die sich von ungesättigten Alkoholen ableiten, gehören 2-Propinyl(meth)acrylat, Allyl(meth)acrylat und Vinyl(meth)acrylat.

Zu den (Meth)acrylate mit 9 bis 30 Kohlenstoffatomen, vorzugsweise 9 bis 24 Kohlenstoffatomen im Alkylrest mit mindestens einer Doppelbindung im Alkylrest gehören insbesondere (Meth)acrylate, die sich von ungesättigten Fettsäuren, Fettalkoholen und Fettsäureamiden ableiten, wie Heptadecenyloyloxy-2-ethyl-(meth)acrylsäureamid, Heptadecan-dien-yloyloxy-2-ethyl-(meth)acrylsäureamid, Heptadecan-trien-yloyloxy-2-ethyl-(meth)acrylsäureamid, Heptadecenyloyloxy-2-ethyl-(meth)acrylsäureamid, (Meth)acryloyloxy-2-ethyl-palmitoleinsäuresäureamid, (Meth)acryloyloxy-2-ethyl-ölsäureamid, (Meth)acryloyloxy-2-ethyl-icosensäureamid, (Meth)acryloyloxy-2-ethyl-cetoleinsäureamid, (Meth)acryloyloxy-2-ethyl-erucasäureamid, (Meth)acryloyloxy-2-ethyl-linolsäureamid, (Meth)acryloyloxy-2-ethyl-linolensäureamid, (Meth)acryloyloxy-2-propyl-palmitoleinsäuresäureamid, (Meth)acryloyloxy-2-propyl-ölsäureamid, (Meth)acryloyloxy-2-propyl-icosensäureamid, (Meth)acryloyloxy-2-propyl-cetoleinsäureamid, (Meth)acryloyloxy-2-propyl-erucasäureamid, (Meth)acryloyloxy-2-propyl-linolsäureamid und (Meth)acryloyloxy-2-propyl-linolensäureamid; (Meth)acryloyloxy-2-hydroxypropyl-linolsäureester, (Meth)acryloyloxy-2-hydroxypropyl-linolensäureester und (Meth)acryloyloxy-2-hydroxypropyl-ölsäureester; Octadecan-dien-yl-(meth)acrylat, Octadecan-trien-yl-(meth)acrylat, Hexadecenyl(meth)acrylat, Octadecenyl(meth)acrylat und Hexadecan-dien-yl-(meth)acrylat.

Weiterhin gehören zu den Meth)acrylate mit 9 bis 30 Kohlenstoffatomen, vorzugsweise 9 bis 24 Kohlenstoffatomen im Alkylrest mit mindestens einer Doppelbindung im Alkylrest zählen insbesondere (Meth)acrylatmonomere der allgemeinen Formel (IV) 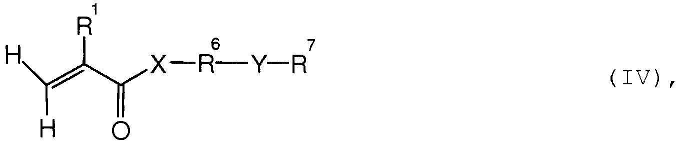 worin R¹ Wasserstoff oder eine Methylgruppe, X Sauerstoff oder eine Gruppe der Formel NR', worin R' Wasserstoff oder ein Rest mit 1 bis 6 Kohlenstoffatomen ist, R⁶ eine Alkylengruppe mit 1 bis 22 Kohlenstoffatomen, Y Sauerstoff, Schwefel oder eine Gruppe der Formel NR" bedeutet, worin R" Wasserstoff oder ein Rest mit 1 bis 6 Kohlenstoffatomen darstellt, und R⁷ ein ungesättigter Rest mit 8 Kohlenstoffatomen und mindestens zwei Doppelbindungen ist. Hinsichtlich bevorzugter Ausführungsformen des Rests R⁶ wird auf die Ausführungen zum Rest R³ verwiesen, da sich diese Reste entsprechen.

Zu den (Meth)acrylatmonomere der allgemeinen Formel (IV) zählen unter anderem 2-[((2-E)Octa-2,7-dienyl)methylamino]ethyl-2-methylprop-2-enoat, 2-[((2-Z)Octa-2,7-dienyl)methylamino]ethyl-2-methylprop-2-enoat, 2-[((3-E)Octa-3,7-dienyl)methylamino]ethyl-2-methylprop-2-enoat, 2-[((4-Z)Octa-4,7-dienyl)methylamino]ethyl-2-methylprop-2-enoat, 2-[(Octa-2,6-dienyl)methylamino]ethyl-2-methylprop-2-enoat, 2-[(Octa-2,4-dienyl)methylamino]ethyl-2-methylprop-2-enoat, 2-[(Octa-3,5-dienyl)methylamino]ethyl-2-methylprop-2-enoat, 2-[((2-E)Octa-2,7-dienyl)methylamino]ethyl-(meth)acrylsäureamid, 2-[((2-Z)Octa-2,7-dienyl)methylamino]ethyl-(meth)acrylsäureamid, 2-[((3-E)Octa-3,7-dienyl)methylamino]ethyl-(meth)acrylsäureamid, 2-[((4-Z)Octa-4,7-dienyl)methylamino]ethyl-(meth)acrylsäureamid, 2-[(Octa-2,6-dienyl)methylamino]ethyl-(meth)acrylsäureamid, 2-[(Octa-2,4-dienyl)methylamino]ethyl-(meth)acrylsäureamid, 2-[(Octa-3,5-dienyl)methylamino]ethyl-(meth)acrylsäureamid, 2-[((2-E)Octa-2,7-dienyl)ethylamino]ethyl-2-methylprop-2-enoat, 2-[((2-Z)Octa-2,7-dienyl)ethylamino]ethyl-2-methylprop-2-enoat, 2-[((3-E)Octa-3,7-dienyl)ethylamino]ethyl-2-methylprop-2-enoat, 2-[((4-Z)Octa-4,7-dienyl)ethylamino]ethyl-2-methylprop-2-enoat, 2-[(Octa-2,6-dienyl)ethylamino]ethyl-2-methylprop-2-enoat, 2-[(Octa-2,4-dienyl)ethylamino]ethyl-2-methylprop-2-enoat, 2-[(Octa-3,5-dienyl)ethylamino]ethyl-2-methylprop-2-enoat, 2-[((2-E)Octa-2,7-dienyl)methylamino]ethyl-prop-2-enoat, 2-[((2-z)Octa-2,7-dienyl)methylamino]ethyl-prop-2-enoat, 2-[((3-E)Octa-3,7-dienyl)methylamino]ethyl-prop-2-enoat, 2-[((4-Z)Octa-4,7-dienyl)methylamino]ethyl-prop-2-enoat, 2-[(Octa-2,6-dienyl)methylamino]ethyl-prop-2-enoat, 2-[(Octa-2,4-dienyl)methylamino]ethyl-prop-2-enoat, 2-[(Octa-3,5-dienyl)methylamino]ethyl-prop-2-enoat, 2-((2-E)Octa-2,7-dienyloxy)ethyl-2-methylprop-2-enoat, 2-((2-Z)Octa-2,7-dienyloxy)ethyl-2-methylprop-2-enoat, 2-((3-E)Octa-3,7-dienyloxy)ethyl-2-methylprop-2-enoat, 2-((4-Z)Octa-4,7-dienyloxy)ethyl-2-methylprop-2-enoat, 2-(Octa-2,6-dienyloxy)ethyl-2-methylprop-2-enoat, 2-(Octa-2,4-dienyloxy)ethyl-2-methylprop-2-enoat, 2-(Octa-3,5-dienyloxy)ethyl-2-methylprop-2-enoat, 2-((2-E)Octa-2,7-dienyloxy)ethyl-prop-2-enoat, 2-((2-Z)Octa-2,7-dienyloxy)ethyl-prop-2-enoat, 2-((3-E)Octa-3,7-dienyloxy)ethyl-prop-2-enoat, 2-((4-Z)Octa-4,7-dienyloxy)ethyl-prop-2-enoat, 2-(Octa-2,6-dienyloxy)ethyl-prop-2-enoat, 2-(Octa-2,4-dienyloxy)ethyl-prop-2-enoat und 2-(Octa-3,5-dienyloxy)ethyl-prop-2-enoat.

Die Edukte gemäß Formel (III) können einzeln oder als Mischung eingesetzt werden.

Die zuvor dargelegten (Meth)acrylate, die als Edukte eingesetzt werden können, sind teilweise kommerziell erhältlich. Weiterhin können diese (Meth)acrylate durch Telomerisierungsreaktionen, durch Umsetzung von Fettsäuren mit Glycidyl(meth)acrylat, durch Veresterungsreaktionen oder Umesterungsreaktionen erhalten werden.

Umsetzungen von Glycidiyl(meth)acrylat mit Fettsäuren werden unter anderem in WO 2006/013061 dargelegt.

Die zuvor dargelegten (Meth)acrylate lassen sich insbesondere durch Verfahren erhalten, in denen Methacrylsäure, Acrylsäure oder eine Mischung derselben, nachfolgend auch als (Meth)acrylsäure abgekürzt, oder ein (Meth)acrylat, insbesondere Methyl(meth)acrylat oder Ethyl(meth)acrylat mit einem Alkohol und/oder einem Amin umgesetzt wird. Umesterungen von Alkoholen mit (Meth)acrylaten oder die Herstellung von (Meth)acrylsäureamiden sind weiterhin in CN 1355161, DE 21 29 425 eingereicht am 14.06.71 beim Deutschen Patentamt mit der Anmeldenummer P 2129425.7, DE 34 23 443 eingereicht am 26.06.84 beim Deutschen Patentamt mit der Anmeldenummer P 3423443.8, EP-A-O 534 666 eingereicht am 16.09.92 beim Europäischen Patentamt mit der Anmeldenummer EP 92308426.3 oder DE 34 30 446 eingereicht am 18.08.84 beim Deutschen Patentamt mit der Anmeldenummer P 3430446.0 dargelegt, wobei die in diesen Druckschriften beschriebenen Reaktionsbedingungen sowie die darin dargelegten Katalysatoren usw. zu Zwecken der Offenbarung in diese Anmeldung eingefügt werden. Weiterhin sind diese Umsetzungen in "Synthesis of Acrylic Esters by Transesterification", J. Haken, 1967 beschrieben.

Das mit der (Meth)acrylsäure oder dem (Meth)acrylat umzusetzende Edukt kann vorteilhaft der Formel (V) entsprechen,

H-X-R⁶-Y-R⁷ (V),

worin X Sauerstoff oder eine Gruppe der Formel NR', worin R' Wasserstoff oder ein Rest mit 1 bis 6 Kohlenstoffatomen ist, R⁶ eine Alkylengruppe mit 1 bis 22 Kohlenstoffatomen, Y Sauerstoff, Schwefel oder eine Gruppe der Formel NR " bedeutet, worin R" Wasserstoff oder ein Rest mit 1 bis 6 Kohlenstoffatomen darstellt, und R' ein mindestens doppelt ungesättigter Rest mit 8 Kohlenstoffatomen ist.

Hinsichtlich der Bedeutung bevorzugter Reste R', R", R⁶, Y und R⁷ wird auf die Beschreibung der Formel (IV) verwiesen.

Zu den bevorzugten Edukten gemäß Formel (V) gehören (Methyl(octa-2,7-dienyl)amino)ethanol, (Ethyl(octa-2,7-dienyl)amino)ethanol, 2-Octa-2,7-dienyloxyethanol, (Methyl(octa-2,7-dienyl)amino)ethylamin, (Methyl(octa-3,7-dienyl)amino)ethanol, (Ethyl(octa-3,7-dienyl)amino)ethanol, 2-Octa-3,7-dienyloxyethanol, (Methyl(octa-3,7-dienyl)amino)ethylamin, (Methyl(octa-4,7-dienyl)amino)ethanol, (Ethyl(octa-4,7-dienyl)amino)ethanol, 2-Octa-4,7-dienyloxyethanol, (Methyl(octa-4,7-dienyl)amino)ethylamin, (Methyl(octa-5,7-dienyl)amino)ethanol, (Ethyl(octa-5,7-dienyl)amino)ethanol, 2-Octa-5,7-dienyloxyethanol, (Methyl(octa-5,7-dienyl)amino)ethylamin, (Methyl(octa-2,6-dienyl)amino)ethanol, (Ethyl(octa-2,6-dienyl)amino)ethanol, 2-Octa-2,6-dienyloxyethanol, (Methyl(octa-2,6-dienyl)amino)ethylamin, (Methyl(octa-2,5-dienyl)amino)ethanol, (Ethyl(octa-2,5-dienyl)amino)ethanol, 2-Octa-2,5-dienyloxyethanol, (Methyl(octa-2,5-dienyl)amino)ethylamin, (Methyl(octa-2,4-dienyl)amino)ethanol, (Ethyl(octa-2,4-dienyl)amino)ethanol, 2-Octa-2,4-dienyloxyethanol, (Methyl(octa-2,4-dienyl)amino)ethylamin, (Methyl(octa-3,6-dienyl)amino)ethanol, (Ethyl(octa-3,6-dienyl)amino)ethanol, 2-Octa-3,6-dienyloxyethanol, (Methyl(octa-3,6-dienyl)amino)ethylamin, (Methyl(octa-3,5-dienyl)amino)ethanol, (Ethyl(octa-3,5-dienyl)amino)ethanol, 2-Octa-3,5-dienyloxyethanol, (Methyl(octa-3,5-dienyl)amino)ethylamin, (Methyl(octa-4,6-dienyl)amino)ethanol, (Ethyl(octa-4,6-dienyl)amino)ethanol, 2-Octa-4,6-dienyloxyethanol und (Methyl(octa-4,6-dienyl)amino)ethylamin. Die Edukte gemäß Formel (II) können einzeln oder als Mischung eingesetzt werden.

Die Edukte gemäß Formel (V) lassen sich unter anderem durch bekannte Verfahren der Telomerisierung von 1,3-Butadien erhalten. Hierbei bedeutet der Begriff "Telomerisierung" die Umsetzung von Verbindungen mit konjugierten Doppelbindungen in Gegenwart von Nukleophilen. Die in den Druckschriften WO 2004/002931 eingereicht am 17.06.2003 beim Europäischen Patentamt mit der Anmeldenummer PCT/EP2003/006356, WO 03/031379 eingereicht am 01.10.2002 mit der Anmeldenummer PCT/EP2002/10971 und WO 02/100803 eingereicht am 04.05.2002 mit der Anmeldenummer PCT/EP2002/04909 dargelegten Verfahren, insbesondere die zur Umsetzung eingesetzten Katalysatoren und die Reaktionsbedingungen, wie zum Beispiel Druck und Temperatur, werden zu Zwecken der Offenbarung in die vorliegende Anmeldung eingefügt.

Bevorzugt kann die Telomerisierung von 1,3-Butadien unter Verwendung von Metallverbindungen, die Metalle der 8. bis 10. Gruppe des Periodensystems der Elemente umfassen, als Katalysator erfolgen, wobei Palladiumverbindungen, insbesondere Palladiumcarbenkomplexe, die in den zuvor dargelegten Druckschriften näher dargelegt sind, besonders bevorzugt eingesetzt werden können.

Als Nukleophil können insbesondere Dialkohole, wie Ethylenglykol, 1,2-Propandiol, 1,3-Propandiol; Diamine, wie Ethylendiamin, N-Methyl-ethylendiamin, N,N'-Dimethylethylendiamin oder Hexamethylendiamin; oder Aminoalkanole, wie Aminoethanol, N-Methylaminoethanol, N-Ethylaminoethanol, Aminopropanol, N-Methylaminopropanol oder N-Ethylaminopropanol eingesetzt werden.

Bei Verwendung von (Meth)acrylsäure als Nukleophil können beispielsweise Octadienyl(meth)acrylate erhalten werden, die als Edukt zur Herstellung vom Monomeren gemäß Formel (I) geeignet sind.

Die Temperatur, bei der die Telomerisationsreaktion ausgeführt wird, liegt zwischen 10 und 180 °C, bevorzugt zwischen 30 und 120 °C, besonders bevorzugt zwischen 40 und 100 °C. Der Reaktionsdruck beträgt 1 bis 300 bar, bevorzugt 1 bis 120 bar, besonders bevorzugt 1 bis 64 bar und ganz besonders bevorzugt 1 bis 20 bar.

Die Herstellung von Isomeren aus Verbindungen, die eine Octa-2,7-dienyl-Gruppe aufweisen, kann durch Isomerisierung der Doppelbindungen erfolgen, die in den Verbindungen mit einer Octa-2,7-dienyl-Gruppe enthalten sind.

Neben dem oder den Edukten gemäß Formel (III) und den zuvor beschriebenen Katalysatoren wird zur Umsetzung Wasserstoff (H₂) und Kohlenmonoxid (CO) eingesetzt. Vorzugsweise kann die Reaktion bei einem Gesamtgasdruck im Bereich von 1 bis 200 bar, besonders bevorzugt im Bereich von 1 bis 150 bar, insbesondere bevorzugt im Bereich von 1 bis 100 bar durchgeführt werden.

Gemäß einem besonderen Aspekt der vorliegenden Erfindung kann der Wasserstoffdruck, bei dem Reaktion durchgeführt wird, größer sein als der Druck des Kohlenmonoxids.

Die Temperatur bei der die Umsetzung des Edukts gemäß Formel (III) mit Wasserstoff und Kohlenmonoxid durchgeführt wird ist an sich nicht kritisch. Besondere Vorteile können insbesondere dadurch erzielt werden, dass die Reaktion bei einer Temperatur im Bereich von 20 bis 250°C, bevorzugt von 40 bis 200°C, besonders bevorzugt im Bereich von 150 bis 160°C durchgeführt wird.

Gemäß einem besonderen Aspekt der vorliegenden Erfindung kann die Reaktion in einem inerten organischen Lösungsmittel durchgeführt werden. Zu diesen Lösungsmitteln zählen beispielsweise aromatische Kohlenwasserstoffe, wie zum Beispiel Toluol oder Xylol, Dioxan, Carbonsäureester, wie zum Beispiel Ethylacetat. Mit besonderem Vorteil kann die Reaktion im Wesentlichen ohne den Einsatz eines inerten organischen Lösungsmittels durchgeführt werden. In diesem Fall bilden insbesondere die Edukte sowie die Liganden das Medium, in welchem die Reaktion ausgeführt wird.

Überraschende Vorteile lassen sich weiterhin durch die Verwendung eines Stabilisators erzielen. Zu den bevorzugten Stabilisatoren gehören unter anderem Hydrochinone, Hydrochinonether, wie Hydrochinonmonomethylether oder Ditert-butylbrenzcatechin, Phenothiazin, Methylenblau oder sterisch gehinderte Phenole, beispielsweise 2,4-dimethyl-6-tert-butylphenol, sind in der Fachwelt weithin bekannt. Diese Verbindungen können einzeln oder in Form von Mischungen eingesetzt werden und sind im Allgemeinen kommerziell erhältlich. Für weitere Details wird auf die gängige Fachliteratur, insbesondere auf das Römpp-Lexikon Chemie; Herausgeber: J. Falbe, M. Regitz; Stuttgart, New York; 10. Auflage (1996); Stichwort "Antioxidantien" und die an dieser Stelle zitierten Literaturstellen verwiesen.

Nachfolgend soll die vorliegende Erfindung anhand eines Beispiels und Vergleichsbeispielen näher erläutert werden, ohne dass hierdurch eine Beschränkung erfolgen soll.

### Beispiel 1 (Herstellungsbeispiel)

In einem 100 ml Parr Autoklaven mit Druckkonstanthaltung aus einer konstant temperierten Bürette wurden 10 mmol Methacrylsäureallylester in 25 ml THF gegeben und bei einem Druck von 10 bar mit einem CO/H₂-Gasgemisch, welches ein molares Verhältnis von 1:1 aufwies, in Gegenwart von 0,1 mol.-% Rh(acac(CO)₂ und 0,2 mol.-% Xantphos, jeweils bezogen auf Methacrylsäureallylester, für eine Reaktionszeit von 20 Stunden bei einer Temperatur von 65°C umgesetzt. Es wurde eine Ausbeute von 90 % erzielt, wobei das Reaktionsgemisch 4-Oxobutylmethacrylat und 2-Methyl-3-oxopropylmethacrylat umfasste. Das Verhältnis von 4-Oxobutylmethacrylat zu 2-Methyl-3-oxopropylmethacrylat betrug 92:8.

### Beispiel 2 (Herstellungsbeispiel)

Das Beispiel 1 wurde im Wesentlichen wiederholt, wobei jedoch mit 0,02 mol.-% Rh(acac(CO)₂ und 0,04 mol.-% Xantphos, jeweils bezogen auf Methacrylsäureallylester, gearbeitet wurde. Die Ausbeute betrug 85%, das Verhältnis von 4-Oxobutylmethacrylat zu 2-Methyl-3-oxopropylmethacrylat 92:8.

### Beispiel 3 (Herstellungsbeispiel)

Das Beispiel 1 wurde im Wesentlichen wiederholt, wobei jedoch bei einem Druck von 20 bar gearbeitet wurde. Die Ausbeute betrug 95%, das Verhältnis von 4-Oxobutylmethacrylat zu 2-Methyl-3-oxopropylmethacrylat 88:12.

### Beispiel 4 (Herstellungsbeispiel)

In einem 100 ml Parr Autoklaven mit Druckkonstanthaltung aus einer konstant temperierten Bürette wurden 50 mmol Methacrylsäureallylester in 25 ml THF gegeben und bei einem Druck von 20 bar mit einem CO/H₂-Gasgemisch, welches ein molares Verhältnis von 1:1 aufwies, in Gegenwart von 0,02 mol.-% (2,5 mg) Rh(acac(CO)₂ und 0,04 mol.-% Biphephos, jeweils bezogen auf Methacrylsäureallylester, für eine Reaktionszeit von 2 Stunden bei einer Temperatur von 65°C umgesetzt. Es wurde eine Ausbeute von 99 % erzielt, wobei das Reaktionsgemisch 4-Oxobutylmethacrylat und 2-Methyl-3-oxopropylmethacrylat umfasste. Das Verhältnis von 4-Oxobutylmethacrylat zu 2-Methyl-3-oxopropylmethacrylat betrug 60:40.

### Beispiel 5 (Herstellungsbeispiel)

Das Beispiel 4 wurde im Wesentlichen wiederholt, wobei jedoch bei einem Druck von 10 bar gearbeitet wurde. Die Ausbeute betrug 99%, das Verhältnis von 4-Oxobutylmethacrylat zu 2-Methyl-3-oxopropylmethacrylat 68:32.

### Beispiel 6 (Herstellungsbeispiel)

Das Beispiel 5 wurde im Wesentlichen wiederholt, wobei jedoch mit 0,02 mol.-% Rh(acac(CO)₂ und 0,12 mol.-% Biphephos, jeweils bezogen auf Methacrylsäureallylester, gearbeitet wurde. Die Ausbeute betrug 99%, das Verhältnis von 4-Oxobutylmethacrylat zu 2-Methyl-3-oxopropylmethacrylat 73:27.

### Beispiel 7 (Herstellungsbeispiel)

In einem 100 ml Autoklaven mit Druckkonstanthaltung aus einer konstant temperierten Bürette wurden 10 mmol Methacrylsäureallylester in 25 ml THF gegeben und bei einem Druck von 10 bar mit einem CO/H₂-Gasgemisch, welches ein molares Verhältnis von 1:1 aufwies, in Gegenwart von 0,1 mol.-% Rh(acac(CO)₂ und 0,2 mol.-% Xantphos, jeweils bezogen auf Methacrylsäureallylester, für eine Reaktionszeit von 20 Stunden bei einer Temperatur von 65°C umgesetzt. Es wurde eine Ausbeute von 90 % erzielt, wobei das Reaktionsgemisch 4-Oxobutylmethacrylat und 2-Methyl-3-oxopropylmethacrylat umfasste. Das Verhältnis von 4-Oxobutylmethacrylat zu 2-Methyl-3-oxopropylmethacrylat betrug 92:8.

### Beispiel 8

In einem mit Stickstoff inertisiertem 3000 ml Büchi-Autoklav (Hersteller: Büchi) mit Druckkonstanthaltung und Gaseintragsrührwerk, wurden 1.03 mmol Biphephos, 0.17 mmol Rh(acac(CO)₂ und 2.08 mmol Bis(2,2,6,6-Tetramethyl-4-Piperidinyl) Sebacate in 1200ml Toluol gelöst und in den Autoklaven eingefüllt. Anschließend wurde der Autoklav mit 5bar eines CO/H2 Gasgemisch beaufschlagt, welches ein molares Verhältnis von 1:1 aufwies und für 60 min auf 80°C geheizt. Der Autoklav wurde auf Raumtemperatur abgekühlt und 3 mol Methacrylsäureallylester, die einen Zusatz von 3,2 mmol 4-Hydroxy TEMPO enthielten, wurde zu der toluolischen Katalysatorlösung in den Autoklaven gepumpt. Die anschließende Umsetzung wurde bei 5 bar des oben beschriebenen Gasgemisches durchgeführt. Die Temperatur wurde in dieser Zeit von 25°C auf 30°C gesteigert. Nach 100 min wurde die Reaktion abgebrochen, da keine Gasaufnahme mehr zu verzeichnen war. Daraufhin wurde dem System eine Lösung aus 1.7 mmol Biphephos, 0.04 mmol Rh(acac(CO)₂, 2.1 mmol Bis(2,2,6,6-Tetramethyl-4-Piperidinyl) Sebacate und 0.6 mmol 4-Hydroxy TEMPO gelöst in 450ml Toluol, über eine Pumpe zudosiert und die Reaktion erneut bei 5 bar Reaktionsdruck des Gasgemisches und bei einer Temperatur von 60°C gestartet. Nach 72 min war die Reaktion beendet. Es wurde eine Ausbeute von 61 % erzielt, wobei das Reaktionsgemisch 4-Oxobutylmethacrylat und 2-Methyl-3-oxopropylmethacrylat umfasste. Das Verhältnis von 4-Oxobutylmethacrylat zu 2-Methyl-3-oxopropylmethacrylat betrug 69:31.

### Beispiel 9

In einem mit Stickstoff inertisiertem 3000 ml Büchi-Autoklav mit Druckkonstanthaltung und Gaseintragsrührwerk, wurden 2.5 mmol Biphephos, 0.17 mmol Rh(acac(CO)₂ und 4.1 mmol Bis(2,2,6,6-Tetramethyl-4-Piperidinyl) Sebacate in 1500ml Toluol gelöst und in den Autoklaven eingefüllt. 3,5 mol Methacrylsäureallylester, die einen Zusatz von 3,2 mmol 4-Hydroxy TEMPO enthielten, wurden anschließend in den Autoklaven überführt. Durch die Druckkonstanthaltung wurde der Reaktionsdruck mit dem in Bespiel 1 beschriebenen Gasgemisch auf 5 bar konstant gehalten. Ausgehend von Raumtemperatur wurde der Autoklav durch einen Doppelmantel mit angeschlossenem 2 kW Thermostaten auf 60°C Reaktionstemperatur erwärmt. Nach 105 min wurde die Reaktion abgebrochen, da keine Gasaufnahme mehr zu verzeichnen war. Es wurde eine Ausbeute von 72 % erzielt, wobei das Reaktionsgemisch 4-Oxobutylmethacrylat und 2-Methyl-3-oxopropylmethacrylat umfasste. Das Verhältnis von 4-Oxobutylmethacrylat zu 2-Methyl-3-oxopropylmethacrylat betrug 70:30.

### Beispiel 10

In einem mit Stickstoff inertisiertem 3000 ml Büchi-Autoklav mit Druckkonstanthaltung und Gaseintragsrührwerk wurden 1.37 mmol Triphenylphosphin, 1.02 mmol Biphephos, 0.17 mmol Rh(acac(CO)₂ und 4.1 mmol Bis(2,2,6,6-Tetramethyl-4-Piperidinyl) Sebacate in 1500ml Toluol gelöst und in den Autoklaven eingefüllt. 3,4 mol Methacrylsäureallylester, die einen Zusatz von 3,2 mmol 4-Hydroxy TEMPO enthielten, wurden anschließend in den Autoklaven überführt. Durch die Druckkonstanthaltung wurde der Reaktionsdruck mit dem in Bespiel 1 beschriebenen Gasgemisch auf 5 bar konstant gehalten. Ausgehend von Raumtemperatur wurde der Autoklav durch einen Doppelmantel mit angeschlossenem 2 kW Thermostaten auf 60°C Reaktionstemperatur erwärmt. Die Temperatur wurde in nach 27 min auf 70°C und nach 49 min auf 75°C angehoben. Nach 120 min wurde die Reaktion abgebrochen, da keine Gasaufnahme mehr zu verzeichnen war. Es wurde eine Ausbeute von 74 % erzielt, wobei das Reaktionsgemisch 4-Oxobutylmethacrylat und 2-Methyl-3-oxopropylmethacrylat umfasste. Das Verhältnis von 4-Oxobutylmethacrylat zu 2-Methyl-3-oxopropylmethacrylat betrug 78:22.

### Beispiel 11

In einem mit Stickstoff inertisiertem 3000 ml Büchi-Autoklav mit Druckkonstanthaltung und Gaseintragsrührwerk, wurden 2.1 mmol Triphenylphosphin, 1.05 mmol Biphephos, 0.17 mmol Rh(acac(CO)₂ und 4.2 mmol Bis(2,2,6,6-Tetramethyl-4-Piperidinyl) Sebacate in 1000 ml Toluol gelöst und in den Autoklaven eingefüllt. 5,5 mol Methacrylsäureallylester, die einen Zusatz von 6.6 mmol 4-Hydroxy TEMPO enthielten, wurden anschließend in den Autoklaven überführt. Durch die Druckkonstanthaltung wurde der Reaktionsdruck mit dem in Bespiel 1 beschriebenen Gasgemisch auf 5 bar konstant gehalten. Ausgehend von Raumtemperatur wurde der Autoklav durch einen Doppelmantel mit angeschlossenem 2 kW Thermostaten auf 60°C Reaktionstemperatur erwärmt. Die Temperatur wurde in nach 15 min auf 70°C und nach 20 min auf 75°C angehoben. Nach 210 min wurde die Reaktion abgebrochen, da keine Gasaufnahme mehr zu verzeichnen war. Es wurde eine Ausbeute von 60 % erzielt, wobei das Reaktionsgemisch 4-Oxobutylmethacrylat und 2-Methyl-3-oxopropylmethacrylat umfasste. Das Verhältnis von 4-Oxobutylmethacrylat zu 2-Methyl-3-oxopropylmethacrylat betrug 82:18.

### Beispiel 12

In einem mit Stickstoff inertisiertem 3000 ml Büchi-Autoklav mit Druckkonstanthaltung und Gaseintragsrührwerk, wurden 2.1 mmol Triphenylphosphin, 1.05 mmol Biphephos, 0.17 mmol Rh(acac(CO)₂ und 4.2 mmol Bis(2,2,6,6-Tetramethyl-4-Piperidinyl) Sebacate in 1000 ml Toluol gelöst und in den Autoklaven eingefüllt. 6,3 mol Methacrylsäureallylester, die einen Zusatz von 6.6 mmol 4-Hydroxy TEMPO enthielten, wurden anschließend in den Autoklaven überführt. Der Autoklav wurde dreimal mit dem Reaktionsgasgemisch auf 6 bar abs. aufgedrückt und anschließend wieder auf 1 bar abs. entspannt.

Durch die Druckkonstanthaltung wurde der Reaktionsdruck mit dem in Bespiel 1 beschriebenen Gasgemisch auf 5 bar konstant gehalten. Ausgehend von Raumtemperatur wurde der Autoklav durch einen Doppelmantel mit angeschlossenem 2 kW Thermostaten auf 75°C Reaktionstemperatur erwärmt. Nach 190 min wurde die Reaktion abgebrochen, da keine Gasaufnahme mehr zu verzeichnen war.

Es wurde eine Ausbeute von 53 % erzielt, wobei das Reaktionsgemisch 4-Oxobutylmethacrylat und 2-Methyl-3-oxopropylmethacrylat umfasste. Das Verhältnis von 4-Oxobutylmethacrylat zu 2-Methyl-3-oxopropylmethacrylat betrug 80:20.

### Beispiel 13

In einem mit Stickstoff inertisiertem 3000 ml Büchi-Autoklav mit Druckkonstanthaltung und Gaseintragsrührwerk, wurden 2.1 mmol Triphenylphosphin, 1.05 mmol Biphephos, 0.17 mmol Rh(acac(CO)₂ und 4.2 mmol Bis(2,2,6,6-Tetramethyl-4-Piperidinyl) Sebacate in 1000 ml Toluol gelöst und in den Autoklaven eingefüllt. 6,4 mol Methacrylsäureallylester, die einen Zusatz von 0.47 mmol 4-Hydroxy TEMPO enthielten, wurden anschließend in den Autoklaven überführt.

Der Autoklav wurde dreimal mit dem Reaktionsgasgemisch auf 6 bar abs. aufgedrückt und anschließend wieder auf 1 bar abs. entspannt.

Durch die Druckkonstanthaltung wurde der Reaktionsdruck mit dem in Bespiel 1 beschriebenen Gasgemisch auf 5 bar konstant gehalten. Ausgehend von Raumtemperatur wurde der Autoklav durch einen Doppelmantel mit angeschlossenem 2 kW Thermostaten auf 75°C Reaktionstemperatur erwärmt. Nach 190 min wurde die Reaktion abgebrochen, da keine Gasaufnahme mehr zu verzeichnen war.

Es wurde eine Ausbeute von 54 % erzielt, wobei das Reaktionsgemisch 4-Oxobutylmethacrylat und 2-Methyl-3-oxopropylmethacrylat umfasste. Das Verhältnis von 4-Oxobutylmethacrylat zu 2-Methyl-3-oxopropylmethacrylat betrug 80:20.

### Beispiel 14

In einem mit Stickstoff inertisiertem 3000 ml Büchi-Autoklav mit Druckkonstanthaltung und Gaseintragsrührwerk, wurden 2.1 mmol Triphenylphosphin, 1.05 mmol Biphephos, 0.17 mmol Rh(acac(CO)₂ und 4.2 mmol Bis(2,2,6,6-Tetramethyl-4-Piperidinyl) Sebacate in 1000 ml Toluol gelöst und in den Autoklaven eingefüllt. 6,8 mol Methacrylsäureallylester, die einen Zusatz von 0.53 mmol 4-Hydroxy TEMPO enthielten, wurden anschließend in den Autoklaven überführt. Der Autoklav wurde dreimal mit dem Reaktionsgasgemisch auf 6 bar abs. aufgedrückt und anschließend wieder auf 1 bar abs. entspannt.

Durch die Druckkonstanthaltung wurde der Reaktionsdruck mit dem in Bespiel 1 beschriebenen Gasgemisch auf 10 bar konstant gehalten. Ausgehend von Raumtemperatur wurde der Autoklav durch einen Doppelmantel mit angeschlossenem 2 kW Thermostaten auf 75°C Reaktionstemperatur erwärmt. Nach 190 min wurde die Reaktion abgebrochen, da keine Gasaufnahme mehr zu verzeichnen war.

Es wurde eine Ausbeute von 61 % erzielt, wobei das Reaktionsgemisch 4-Oxobutylmethacrylat und 2-Methyl-3-oxopropylmethacrylat umfasste. Das Verhältnis von 4-Oxobutylmethacrylat zu 2-Methyl-3-oxopropylmethacrylat betrug 80:20.

### Beispiel 15

In einem mit Stickstoff inertisiertem 3000 ml Büchi-Autoklav mit Druckkonstanthaltung und Gaseintragsrührwerk, wurden 2.1 mmol Triphenylphosphin, 1.05 mmol Biphephos, 0.17 mmol Rh(acac(CO)₂ und 4.2 mmol Bis(2,2,6,6-Tetramethyl-4-Piperidinyl) Sebacate in 1000 ml Toluol gelöst und in den Autoklaven eingefüllt. 6,8 mol Methacrylsäureallylester, die einen Zusatz von 0.53 mmol 4-Hydroxy TEMPO enthielten, wurden anschließend in den Autoklaven überführt. Der Autoklav wurde dreimal mit dem Reaktionsgasgemisch auf 6 bar abs. aufgedrückt und anschließend wieder auf 1 bar abs. entspannt.

Durch die Druckkonstanthaltung wurde der Reaktionsdruck mit dem in Bespiel 1 beschriebenen Gasgemisch auf 5 bar konstant gehalten. Ausgehend von Raumtemperatur wurde der Autoklav durch einen Doppelmantel mit angeschlossenem 2 kW Thermostaten auf 75°C Reaktionstemperatur erwärmt. Nach 185 min wurde die Reaktion abgebrochen, da keine Gasaufnahme mehr zu verzeichnen war. Es wurde eine Ausbeute von 57 % erzielt, wobei das Reaktionsgemisch 4-Oxobutylmethacrylat und 2-Methyl-3-oxopropylmethacrylat umfasste. Das Verhältnis von 4-Oxobutylmethacrylat zu 2-Methyl-3-oxopropylmethacrylat betrug 80:20.

## Patentansprüche

1. Verfahren zur Herstellung eines Monomers der allgemeinen Formel (I) 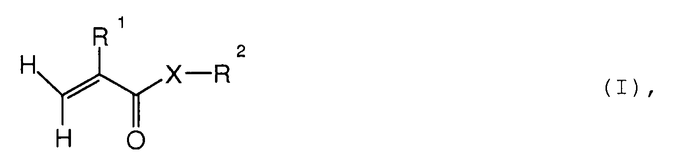 worin R¹ Wasserstoff oder eine Methylgruppe, X Sauerstoff oder eine Gruppe der Formel NR', worin R' Wasserstoff oder ein Rest mit 1 bis 6 Kohlenstoffatomen ist, R² Alkylgruppe mit 3 oder 4 Kohlenstoffatomen und einer Aldehydgruppe ist, **dadurch gekennzeichnet, dass** ein Edukt der Formel (III) 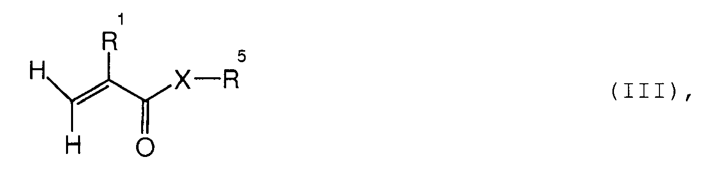 worin R¹ Wasserstoff oder eine Methylgruppe, X Sauerstoff oder eine Gruppe der Formel NR', worin R' Wasserstoff oder ein Rest mit 1 bis 6 Kohlenstoffatomen ist, R⁵ ein ungesättigter Rest mit mindestens einer Doppelbindung und 2 bis 30 Kohlenstoffatomen ist, mit Kohlenmonoxid und Wasserstoff in Gegenwart eines Katalysators umgesetzt wird.

2. Verfahren gemäß Anspruch 1, **dadurch gekennzeichnet, dass** der Katalysator Rhodium, Iridium, Palladium und/oder Cobalt umfasst.

3. Verfahren gemäß Anspruch 1 oder 2, **dadurch gekennzeichnet, dass** der Katalysator ein Komplex ist, der eine phosphorhaltige Verbindung als Ligand umfasst.

4. Verfahren gemäß einem der Ansprüche 1 bis 3, **dadurch gekennzeichnet, dass** die Reaktion bei einer Temperatur im Bereich von 20 bis 250°C, bevorzugt von 40 bis 200°C, besonders bevorzugt im Bereich von 150 bis 160°C durchgeführt wird.

5. Verfahren gemäß einem der Ansprüche 1 bis 4, **dadurch gekennzeichnet, dass** die Reaktion bei einem Gesamtgasdruck im Bereich von 1 bis 200 bar, besonders bevorzugt im Bereich von 1 bis 150 bar, insbesondere bevorzugt im Bereich von 1 bis 100 bar durchgeführt wird.

6. Verfahren gemäß einem der Ansprüche 1 bis 5, **dadurch gekennzeichnet, dass** der Wasserstoffdruck, bei dem Reaktion durchgeführt wird, größer ist als der Druck des Kohlenmonoxids.

7. Verfahren gemäß einem der Ansprüche 1 bis 6, **dadurch gekennzeichnet, dass** die als Ligand eingesetzte phosphorhaltige Verbindung im Überschuss zum Metall eingesetzt wird und das Verhältnis von Metall zu Ligand vorzugsweise im Bereich von 1:1 bis 1:1000, besonders bevorzugt im Bereich von 1:2 und 1:200 liegt.

8. Verfahren gemäß einem der Ansprüche 1 bis 7, **dadurch gekennzeichnet, dass** die Reaktion in einem inerten organischen Lösungsmittel durchgeführt wird.

9. Verfahren gemäß einem der Ansprüche 1 bis 8, **dadurch gekennzeichnet, dass** die Reaktion im Wesentlichen ohne den Einsatz eines inerten organischen Lösungsmittels durchgeführt wird.

10. Verfahren gemäß einem der Ansprüche 1 bis 9, **dadurch gekennzeichnet, dass** die Reaktion in Gegenwart eines Stabilisators durchgeführt wird.

## Claims

1. Process for preparing a monomer of the general formula (I) 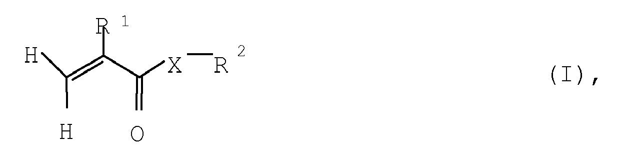 in which R¹ is hydrogen or a methyl group, X is oxygen or a group of the formula NR' in which R' is hydrogen or a radical having 1 to 6 carbon atoms, and R² is an alkyl group having 3 or 4 carbon atoms and an aldehyde group,
**characterized in that** a reactant of the formula (III) 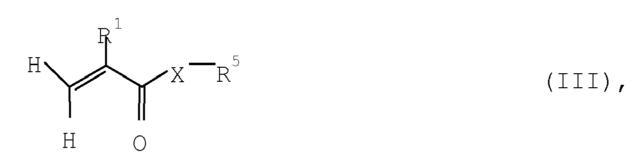 in which R1 is hydrogen or a methyl group, X is oxygen or a group of the formula NR' in which R' is hydrogen or a radical having 1 to 6 carbon atoms, and R⁵ is an unsaturated radical having at least one double bond and 2 to 30 carbon atoms, is reacted with carbon monoxide and hydrogen in the presence of a catalyst.

2. Process according to Claim 1, **characterized in that** the catalyst comprises rhodium, iridium, palladium and/or cobalt.

3. Process according to Claim 1 or 2, **characterized in that** the catalyst is a complex which comprises a phosphorus compound as ligand.

4. Process according to any of Claims 1 to 3, **characterized in that** the reaction is carried out at a temperature in the range from 20 to 250°C, preferably from 40 to 200°C, more preferably in the range from 150 to 160°C.

5. Process according to any of Claims 1 to 4, **characterized in that** the reaction is carried out at an overall gas pressure in the range from 1 to 200 bar, more preferably in the range from 1 to 150 bar, very preferably in the range from 1 to 100 bar.

6. Process according to any of Claims 1 to 5, **characterized in that** the hydrogen pressure at which reaction is carried out is greater than the pressure of the carbon monoxide.

7. Process according to any of Claims 1 to 6, **characterized in that** the phosphorus compound employed as ligand is used in excess over the metal and the ratio of metal to ligand is preferably in the range from 1:1 to 1:1000, more preferably in the range from 1:2 to 1:200.

8. Process according to any of Claims 1 to 7, **characterized in that** the reaction is carried out in an inert organic solvent.

9. Process according to any of Claims 1 to 8, **characterized in that** the reaction is carried out substantially without the use of an inert organic solvent.

10. Process according to any of Claims 1 to 9, **characterized in that** the reaction is carried out in the presence of a stabilizer.

## Revendications

1. Procédé pour la préparation d'un monomère de formule générale (I) 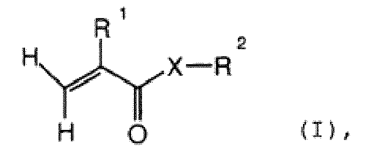 dans laquelle R¹ représente hydrogène ou un groupe méthyle, X représente oxygène ou un groupe de formule NR', dans laquelle R' représente hydrogène ou un radical comprenant 1 à 6 atomes de carbone, R² représente un groupe alkyle comprenant 3 ou 4 atomes de carbone et un groupe aldéhyde, **caractérisé en ce qu'**un produit de départ de formule (III) 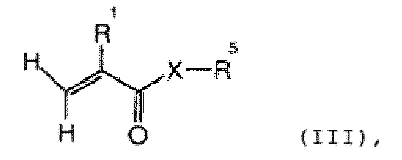 dans laquelle R¹ représente hydrogène ou un groupe méthyle, X représente oxygène ou un groupe de formule NR', dans laquelle R' représente hydrogène ou un radical comprenant 1 à 6 atomes de carbone, R⁵ représente un radical insaturé comprenant au moins une double liaison et 2 à 30 atomes de carbone est transformé avec du monoxyde de carbone et de l'hydrogène en présence d'un catalyseur.

2. Procédé selon la revendication 1, **caractérisé en ce que** le catalyseur comprend du rhodium, de l'iridium, du palladium et/ou du cobalt.

3. Procédé selon la revendication 1 ou 2, **caractérisé en ce que** le catalyseur est un complexe qui comprend un composé contenant du phosphore comme ligand.

4. Procédé selon l'une quelconque des revendications 1 à 3, **caractérisé en ce que** la réaction est réalisée à une température dans la plage de 20 à 250°C, de préférence de 40 à 200°C, de manière particulièrement préférée dans la plage de 150 à 160°C.

5. Procédé selon l'une quelconque des revendications 1 à 4, **caractérisé en ce que** la réaction est réalisée à une pression gazeuse totale dans la plage de 1 à 200 bars, de manière particulièrement préférée dans la plage de 1 à 150 bars, en particulier de préférence dans la plage de 1 à 100 bars.

6. Procédé selon l'une quelconque des revendications 1 à 5, **caractérisé en ce que** la pression d'hydrogène, à laquelle la réaction est réalisée, est supérieure à la pression du monoxyde de carbone.

7. Procédé selon l'une quelconque des revendications 1 à 6, **caractérisé en ce que** le composé contenant du phosphore utilisé comme ligand est utilisé en excès par rapport au métal et le rapport de métal à ligand se situe de préférence dans la plage de 1:1 à 1:1000, de manière particulièrement préférée dans la plage de 1:2 à 1:200.

8. Procédé selon l'une quelconque des revendications 1 à 7, **caractérisé en ce que** la réaction est réalisée dans un solvant organique inerte.

9. Procédé selon l'une quelconque des revendications 1 à 8, **caractérisé en ce que** la réaction est réalisée sensiblement sans utilisation d'un solvant organique inerte.

10. Procédé selon l'une quelconque des revendications 1 à 9, **caractérisé en ce que** la réaction est réalisée en présence d'un stabilisant.
